# EUROPEAN PATENT APPLICATION

(11) **EP 4 764 577 A1**
(43) Date of publication of application: **24.06.2026**
(21) Application number: 24221665.3
(22) Date of filing: 19.12.2024
(51) Int. Cl.: G01R 33/54, A61B 5/055, G06N 3/04

(54) **INTER-SCAN TIME INTERVALS IN MAGNETIC RESONANCE IMAGING**

(71) Applicant: Koninklijke Philips N.V., 5656 AE Eindhoven (NL)
(72) Inventor: NAUTS, Sanne, Eindhoven (NL); WUELBERN, Jan Hendrik, Eindhoven (NL); HELLE, Michael Günter, 5656AG Eindhoven (NL); FRERKING, Lena Christina, Eindhoven (NL); WIEMKER, Rafael, Eindhoven (NL); VAN EE, Raymond, Eindhoven (NL); HEUVELINK, Annerieke, Eindhoven (NL)
(74) Representative: Philips Intellectual Property & Standards

(57) **Abstract**

Disclosed herein is a method of magnetic resonance imaging. The method comprises receiving (200) a sequence of magnetic resonance imaging scans (122) to be performed on a single examination subject (318) during a continuous time interval for a magnetic resonance imaging system (302).

The method further comprises receiving (202) metadata (124) descriptive of the single examination subject and the magnetic resonance imaging system. The method further comprises receiving (204) a set of inter-scan time intervals (126) for time intervals between the sequence of magnetic resonance imaging scans in response to inputting the metadata and the sequence of magnetic resonance imaging scans into a trained artificial intelligence module (132). The set of inter-scan time intervals comprises: paired time intervals (128) and confidence levels (130) of the time intervals. The method further comprises providing (206) the set of inter-scan time intervals as control data for the magnetic resonance imaging system.

## Description

### FIELD OF INVENTION

The invention relates to magnetic resonance imaging, in particular to easing mental stress during magnetic resonance imaging.

### BACKGROUND OF INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. A combination of radio-frequency signals and magnetic gradients are used to encode the nuclear spins. The nuclear spins will emit radio- frequency signal in response to this encoding. These radio-frequency signals may be sampled and stored digitally. These samples are in samples in k-space and are referred to herein as k-space data. The k-space data may be Fourier transformed into a magnetic resonance image.

A difficulty is that it can take a considerable amount of time to acquire the k-space data. Movement of the subject during the acquisition of k-space data can corrupt the magnetic resonance image or cause motion artifacts.

United States patent application publication US2018014803A1 discloses a method for determining a current remaining time during a measurement for the purpose of medical imaging, an evaluation unit, a medical imaging device, and a computer program product are provided. The method provides for a permitted operating range and an item of extrapolation information regarding the critical event to be provided. The current remaining time is determined by an evaluation unit based on the permitted operating range and the item of extrapolation information.

### SUMMARY OF INVENTION

The invention provides for a method of magnetic resonance imaging, a computer program product, and a medical system in the independent claims. Examples are given in the dependent claims.

In one aspect a method of magnetic resonance imaging is disclosed. The method comprises receiving a sequence of magnetic resonance imaging scans to be performed on a single examination subject during a continuous time interval for a magnetic resonance imaging system. The method further comprises receiving metadata descriptive of the subject and the magnetic resonance imaging system. The method further comprises receiving a set of inter-scan time intervals for time intervals between the magnetic resonance imaging scans in response to inputting the metadata and the magnetic resonance imaging scans into a trained artificial intelligence module. The set of inter-scan time intervals comprises: paired time intervals and confidence levels of the time intervals. The method further comprises providing the set of inter-scan time intervals as control data for the magnetic resonance imaging system.

In another aspect, a computer program product is disclosed. The computer program product comprises a computer-readable storage medium having machine-executable instructions embodied therewith. The machine-executable instructions are configured to implement an example of the method of magnetic resonance imaging.

In another aspect, a medical system is disclosed. The medical system comprises a memory storing machine-executable instructions and a trained artificial intelligence module. The medical system further comprises a computation system. Execution of the machine-executable instructions causes the computational system to receive a sequence of magnetic resonance imaging scans to be performed on a single examination subject during a continuous time interval for a magnetic resonance imaging system. Execution of the machine-executable instructions further causes the computational system to receive metadata descriptive of the subject and the magnetic resonance imaging system.

Execution of the machine-executable instructions further causes the computational system to receive a set of inter-scan time intervals for time intervals between the magnetic resonance imaging scans in response to inputting the metadata and the magnetic resonance imaging scans into the trained artificial intelligence module. The set of inter-scan time intervals comprises paired time intervals and confidence levels of the time intervals. Execution of the machine-executable instructions further causes the computational system to provide the set of inter-scan time intervals as control data for the magnetic resonance imaging system.

### BRIEF DESCRIPTION OF THE DRAWINGS

In the following examples of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates a further example of a medical system.
Fig. 4 shows a flow chart which illustrates a method of using the medical system of Fig. 3.
Fig. 5 illustrates a cloud-based example of a medical system.
Fig. 6 illustrates an exemplary neural network.
Fig. 7 illustrates an example of a subject display.

### DETAILED DESCRIPTION OF EMBODIMENTS

Like numbered elements in these figures are either equivalent elements or perform the same function. Elements which have been discussed previously will not necessarily be discussed in later figures if the function is equivalent.

In one example there is a method of magnetic resonance imaging. The method comprises receiving a sequence of magnetic resonance imaging scans to be performed on a single examination subject during a continuous time interval for a magnetic resonance imaging system. A continuous time interval may for example be a visit to an imaging system or center. The sequence of magnetic resonance imaging scans may for example be a series of magnetic resonance imaging scans that a physician orders for a particular subject. The method further comprises receiving metadata descriptive of the subject and the magnetic resonance imaging system. The metadata descriptive of the subject may contain such things as the health state of the subject, the age of the subject, the weight of the subject and other factors which could affect the magnetic resonance imaging of the subject. The metadata descriptive of the magnetic resonance imaging system may include such things as an identifier of the particular magnetic resonance imaging system, a location of the system, a type of magnetic resonance imaging system and other details.

The method further comprises receiving a set of inter-scan time intervals for time intervals between the sequence of magnetic resonance imaging scans in response to inputting the metadata and the sequence of magnetic resonance imaging scans into a trained artificial intelligence module. The set of inter-scan time intervals comprises paired sets of time intervals and confidence levels of the time intervals.

When a sequence of magnetic resonance imaging scans is performed using the magnetic resonance imaging system, there may be some setup time, time to adjust the position of the subject, and other tasks which are performed and cause a time interval where the magnetic resonance imaging system is not active. This time when the magnetic resonance imaging system is not being used to acquire k-space data may be considered being comprised in or forming the time intervals. The confidence levels of the time intervals may be basically a probability or a number related to a probability of the time interval being correct. For example, if the confidence level of the time interval was low then it would be likely that the time interval from the trained artificial intelligence module may likely be an error. If the confidence level is high, then the particular time interval would be known with a relatively good degree of confidence.

The method further comprises providing the set of inter-scan time intervals as control data for the magnetic resonance imaging system. This example may be beneficial because a knowledge of the inter-scan time intervals could for example be used for managing resources of the magnetic resonance imaging system at the times when the magnetic resonance imaging system is inactive or not acquiring k-space data.

In another example, the method further comprises acquiring multiple groups of k-space data by sequentially controlling the magnetic resonance imaging system according to the sequence of magnetic resonance imaging scans. For example, each of the magnetic resonance imaging scans may have one or more pulse sequences, which are used to control the magnetic resonance imaging system to acquire the k-space data. The method further comprises displaying an inter-scan progress indicator controlled by the control data to the single examination subject. The inter-scan progress indicator is configured for displaying a time tracker of a current inter-scan time interval of the set of inter-scan time intervals. This is done using a subject display.

The inter-scan progress indicator is modified using the confidence level of the current inter-scan time interval. This example may be beneficial because it may enable the subject to concentrate to find a comfortable position or resume a previous position before acquisition of the k-space data begins again. This may have the effect of greatly reducing motion artifacts in the resulting magnetic resonance images. In many magnetic resonance imaging systems, the subject is inserted into the bore of a magnet. The subjects typically have a minimal amount of space and during the magnetic resonance imaging examination the subject is required to stay still. To assist the subject to stay still, typically during the acquisition of the k-space data modern magnetic resonance imaging systems will display a progress indicator to indicate how much time is remaining on a particular magnetic resonance imaging scan or acquisition of k-space data. This helps the subject to remain still and is mentally calming.

A difficulty is that the time interval between the acquisitions of k-space data is typically unknown. The progress indicator may for example show the progress of a particular acquisition of k-space data and then there may be a long pause or delay as the technologist is preparing the magnetic resonance imaging system for the next scan.

As the subject is waiting for the next magnetic resonance imaging scan to start, the progress indicator is no longer shown or is stopped in current systems. The subject may then not have an accurate idea of when the acquisition of k-space data will resume. It is critical in magnetic resonance imaging examinations to remain as still as possible. If the subject knows when the acquisition of k-space data will resume it may be possible for the subject to better hold her/his position during the pause or to find a more comfortable position. Displaying the time tracker for a current inter-scan time interval may result in the subsequently acquired k-space data having less subject motion and thereby having fewer artifacts and may be of higher quality.

In another example, the inter-scan progress indicator is modified using the confidence level of the current inter-scan time interval by performing any one of the following: increasing the duration of the current inter-scan time interval and displaying the inter-scan progress indicator with an additional progress indicator. The additional progress indicator comprises an additional time buffer scaled according to the confidence level of the current inter-scan time interval. The additional progress indicator is configured to provide a countdown after the inter-scan progress indicator has finished its countdown. In both of these cases the duration of the current inter-scan time interval or the additional time buffer can be scaled according to the confidence level.

For example, if the confidence level is very high, then there is less need for an additional time buffer or expanding the duration. Conversely, if there is a low degree of confidence in the time interval, then a larger duration or buffer can be used. This may additionally help to provide a subject with an accurate estimate of when the acquisition of k-space data will resume. As was explained above, this may assist in reducing motion artifacts in the resulting magnetic resonance images because the subject was able to hold the prior position or to find a more comfortable position in time.

In another example, the method further comprises monitoring a status of the magnetic resonance imaging system during performance of the sequence of magnetic resonance imaging scans. The status of the magnetic resonance imaging system may for example encompass if the magnetic resonance imaging scans are being performed properly or even if there are distractions or other events which are preventing the magnetic resonance imaging scans from being performed in a timely fashion. This may for example be useful in further refining the determination of the set of inter-scan time intervals.

In another example, the method further comprises detecting a delay during performance of the sequence of magnetic resonance imaging scans using the status of the magnetic resonance imaging system. This for example may be a delay that has already occurred. The method further comprises updating the metadata using the detected delay. The method further comprises updating the set of inter-scan time intervals by inputting the updated metadata and the magnetic resonance imaging scans into the trained artificial intelligence module. The method further comprises updating the inter-scan progress indicator with the updated set of inter-scan time intervals.

During the performance of the sequence of magnetic resonance imaging scans there may be delays or problems which cause one of the sequence of magnetic resonance imaging scans to be delayed itself or even an interval in between them to be delayed. This, for example, may be encoded in some examples in the metadata and used to update the predication. For example, if there has been a delay in between two of the scans this may, in some instances, affect other scans and they may be more likely to have a delay or pause that was unintentional. This may provide for a better estimate of the inter-scan time intervals and thereby increase the ability of the subject to remain still once the acquisition of k-space data resumes because the subject was either able to maintain a previous position or find a more comfortable position.

In another example the method further comprises determining a set of actual inter-scan time intervals using the status of the magnetic resonance imaging system. For example, during the performance of the sequence of magnetic resonance imaging scans the status can be monitored to determine how long the inter-scan time intervals were. The method further comprises constructing training data using the sequence of magnetic resonance imaging scans and the metadata as trial data and using the set of actual inter-scan time intervals as ground truth data. The method further comprises training the trained artificial intelligence module using the trained data further.

In this example, the set of actual inter-scan time intervals may be used to improve or localize the artificial intelligence module. For example, during an initial deployment the artificial intelligence module could be provided to different sites uniformly. As the artificial intelligence module is used at a particular site or magnetic resonance imaging system, the module gradually becomes adapted to the particular magnetic resonance imaging system, users, and protocols that are used there. This may provide for a greater reduction in the stress level of the subject as the predictions of the inter-scan time intervals are more accurate.

In another example, monitoring of the magnetic resonance imaging system is performed using an audio system to monitor gradient noise of the magnetic resonance imaging system. For example, a microphone in the same room as the magnetic resonance imaging system could record audio signals. The gradient noise is quite large in magnetic resonance imaging systems and would provide a means of detecting when the magnetic resonance imaging system is actually being used without having to make any adaptions or changes to the magnetic resonance imaging itself. This may for example be useful in adding examples to older magnetic resonance imaging systems or from different companies where the software is not accessible or it is not desired to modify it.

In another example, the monitoring of the magnetic resonance imaging system is performed by monitoring a push-to-talk control on an intercom system. For example, if there are a lot of difficulties in the magnetic resonance imaging scan, there may be more communication between the operator of the magnetic resonance imaging system and the subject being imaged. Even without monitoring the audio directly, monitoring the push-to-talk control may provide information about the status of the magnetic resonance imaging system.

In another example, the monitoring of the magnetic resonance imaging system is performed by using the audio system to monitor communication of the operator and potentially the subject. For example, the amount of communication between the two could be monitored. In other examples, a speech-to-text system could be used to extract text or other information from the audio communication between the two people. This may, for example, be useful in then classifying the text or delay using a large language model.

In another example, the monitoring of the magnetic resonance imaging system is performed by determining the status using a local controller of the magnetic resonance imaging system. The local controller may for example be the computer which controls the magnetic resonance imaging system. In some examples, the software and APIs of the system may be available directly and the controller itself can provide the status. This may for example provide information about what scans are being performed as well as timestamps so the start and end times of various scans and therefore the intervals between the scans is known to a high degree of accuracy.

In another example, the monitoring of the magnetic resonance imaging system is performed by determining the status using a screen capture of the display of the local controller. Often times in medical imaging systems, a screen replicator may be used to transmit the display to a remote operator. As the software of the system is not accessible directly, a screen capture could be used and an OCR of the screen or other recognition system could be used to extract a status from a capture of the display. This may provide another way of monitoring the magnetic resonance imaging system and determining when the scanning starts and stops as well as the intervals between them without modifying the system.

In another example, the monitoring of the magnetic resonance imaging system is performed by monitoring clicks or usage of a user interface of the magnetic resonance imaging system. For example, monitoring the usage of the local controller or graphical user interface for the local controller. This may provide extremely detailed information about the status of the magnetic resonance imaging system, although this would require access to the software.

In another example, the monitoring of the magnetic resonance imaging system comprises determining the status using a video feed of the magnetic resonance imaging system and/or control room of the magnetic resonance imaging system. For example, there may be a camera which images the room in which the imaging takes place or the control room and a neural network could be used to identify the location of the people in these rooms. It may tell such things as if the subject is actually in the magnetic resonance imaging system or it may also indicate the position and activity of the operator in the control room. For example, if the operator is particularly stationary or is moving around more, this may indicate a change in the status of the magnetic resonance imaging system.

In another example, the trained artificial intelligence module comprises an input layer configured for receiving an encoded input data descriptive of the sequence of magnetic resonance imaging scans and the metadata. The trained artificial intelligence module further comprises multiple fully connected layers connected to the input layer. The trained artificial intelligence module further comprises an output layer that is connected to a last layer of the fully connected layers. The output layer is configured to output encoded output data descriptive of the inter-scan time intervals. For example, it may be encoded to output the delays between the different scans as well as the confidence interval for each of these.

This example may be beneficial because it provides a straightforward but effective means of providing the trained artificial intelligence module. The trained artificial intelligence module may for example be trained by providing it with training data. During the course of using the magnetic resonance imaging system, the metadata for a particular sequence of scans, as well as the actual time intervals, may be recorded. The sequence of magnetic resonance imaging scans and the metadata are then input into the trained artificial intelligence module as trial data and then the actual measured delays or time intervals in between the scans is used as the ground truth data. A technique such as deep learning may be used for training the trained artificial intelligence module.

In other examples, a variety of other types of artificial intelligence may also be used to implement this. For example, the trained artificial intelligence module may also comprise a large language model, a recurrent neural network with a long-short term memory, a statistical learning model, a transform model, a machine learning module and model in general, a support vector regressor, a stochastic gradient descent model, a gradient boosted trees model, and combinations thereof. As with the other model detailed above, the same type of training data may in many cases be used for training one of these models.

In another example, the metadata further comprises any one of the following: a designated operator of the magnetic resonance imaging system, a clinical identifier, for example to identify a particular clinic or operator of the magnetic resonance imaging system, a department identifier to perhaps identify different groups which may be operating the magnetic resonance imaging system, the clicks entered on a control interface of a magnetic resonance imaging system before beginning the sequence, various types of sensor data such as humidity, temperature, ambient noise, or other data may be used. In other examples, the metadata may further comprise camera data, modifications made manually to the pulse sequences, and then various combinations as described here above. All of this data may for example be useful in modeling the inter-scan time intervals.

In another example, the method further comprises determining a total examination time using the sequence of magnetic resonance imaging scans and the control data. The control data contributes the inter-scan time intervals to the total examination time. The total examination time is adjusted further using the confidence levels of the inter-scan time intervals. The method further comprises scheduling the continuous time intervals for the total examination time using a scheduling application. This example may be beneficial because the predictions are then used to provide a better estimate of the total time for the sequence of magnetic resonance imaging scans, which is then used for scheduling the usage of the machine. This may result in a higher usage rate and reduce costs in operating the magnetic resonance imaging system.

In addition, one may use the total examination time for other tasks. For example, one may use the estimate to alert or warn an operator of the subject that the magnetic resonance imaging scans could possibly go over time. Or schedule a subsequent magnetic resonance imaging scan.

In another example, the method further comprises determining a cumulative time for the sequence of magnetic resonance imaging scans using the set of inter-scan time intervals. For example, more or less, the time during which the system would be acquiring k-space data or executing pulse sequences is known. The knowledge of these time intervals could then be used to be added to this and determine the cumulative time. The method further comprises determining a cumulative confidence level using the confidence levels of the inter-scan time intervals. This cumulative confidence level could be calculated in different ways. For example, the lowest confidence level could be selected. In other cases the confidence level could be weighted average using the corresponding time intervals.

In any case, this cumulative confidence level may be used as a confidence level of the cumulative time, which was directly described as being calculated above. The method further comprises selecting media content from a media database to fill the cumulative time using the cumulative time and the cumulative confidence level. The method further comprises providing the media content to the subject during performance of the magnetic resonance imaging scans using the subject media player. This example may be beneficial because if the subject is using the media content during the magnetic resonance imaging scan and it ends, for example, too early, then the subject may become restless. It may therefore be beneficial to schedule the media content to optimally fill the cumulative time.

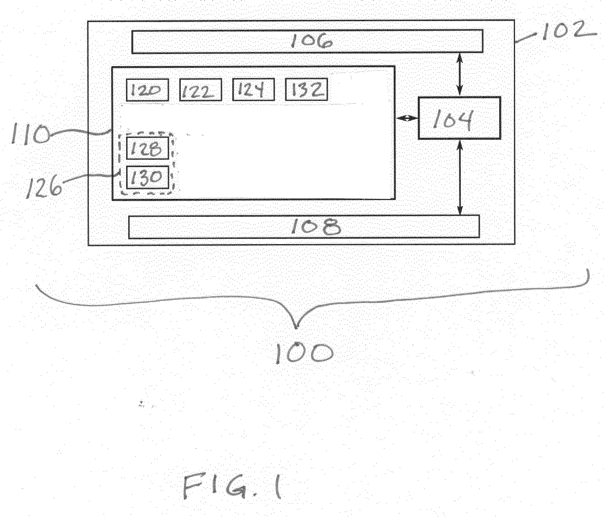

Fig. 1 illustrates an example of a medical system 100 that comprises a computer 102. In some instances, the computer 102 may be a remote server or cloud-based computer or virtual machine. In other cases, the computer 102 may be a local controller, for example, for a magnetic resonance imaging system. The computer 102 is shown as comprising a computational system 104. The computer 102 is intended to represent one or more computers or computing devices and the computational system 104 is intended to represent one or more computational systems or computing cores located at one or more locations. The computational system 104 is shown as being in communication with an optional hardware interface 106 and/or network interface. The interface 106 could for example be used for exchanging data with a network or it could be used for controlling other components of the medical system 100 if they are present. For example, if the computer 102 were a local controller, the optional hardware interface 106 could be used for controlling a magnetic resonance imaging system.

The computational system 104 is further shown as being in communication with an optional user interface 108 that may enable an operator to control the operation and function of the medical system 100. The computational system 104 is additionally shown as being in communication with a memory 110. The memory 110 is intended to represent various types of memory which may be accessible to the computational system 104. In some examples, the memory 110 is a non-transitory storage medium.

The memory 110 is shown as containing machine-executable instructions 120. The machine-executable instructions 120 may enable the computational system 104 to perform such tasks as data analysis, image manipulation or reconstruction, and possibly controlling other components such as a magnetic resonance imaging system. The memory 110 is further shown as containing a sequence of magnetic resonance imaging scans 122. This may for example be a specification of various pulse sequences and anatomical regions to measure in a subject. The memory 110 is further shown as storing metadata 124. This may contain such data as information about the subject and a particular magnetic resonance imaging system. Various other data could also be included in the metadata such as a current status of the magnetic resonance imaging scans.

The memory 110 is further shown as containing a set of inter-scan time intervals 126. The set of inter-scan time intervals 126 comprises paired time intervals 128 and confidence levels 130. The time intervals 128 represent a time interval or predicted time interval between two of the magnetic resonance imaging scans. The confidence levels are a rating of how accurate or how confident the time interval 128 is. The memory 110 is further shown as containing a trained artificial intelligence module 132. The trained artificial intelligence module 132 takes the metadata and the sequence of magnetic resonance imaging scans 122 as input and then outputs the set of inter-scan time intervals 126. For example, the trained artificial intelligence module could be a neural network with fully connected layers. In this case, the sequence of magnetic resonance imaging scans 122 can be input as coded data and, likewise, the set of inter-scan time intervals 126, with its time intervals 128 and confidence levels 130, could be encoded also.

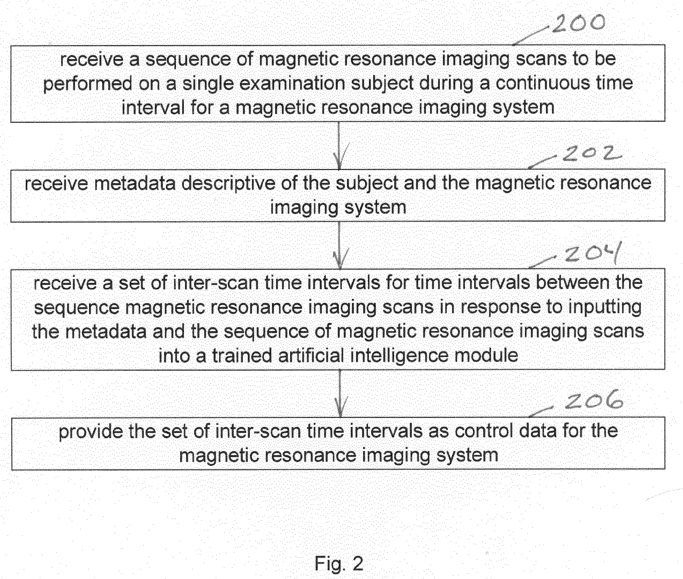

Fig. 2 shows a flowchart which illustrates a method of operating the medical system 100 of Fig. 1. In step 200, the sequence of magnetic resonance imaging scans 122 is received. The sequence of magnetic resonance imaging scans 122 is to be performed on a single examination subject during a continuous time interval or single sitting using a particular magnetic resonance imaging system. In step 202, the metadata 124 is received. The metadata is descriptive of the single examination subject and the magnetic resonance imaging system. In step 204, the set of inter-scan time intervals 126 are received in response to inputting the sequence of magnetic resonance imaging scans 122 and the metadata 124 into the trained artificial intelligence module 132. The set of inter-scan time intervals 126 comprises pairs of time intervals 128 and confidence levels 130. The time intervals 128 are descriptive of a time interval that elapses between two of the magnetic resonance imaging scans 122. It should be noted that the time intervals 128 can be described in several ways. In one instance the time interval 128 is a prediction of a particular duration or time and the confidence level 130 is a rating of how accurate this may or may not be. In other examples the time intervals 128 may be a time range itself. For example, in the first case, it may be two minutes and in the second case it may be between one minute and three minutes. So depending upon the example, the time intervals 128 may be encoded differently.

In step 206 the set of inter-scan time intervals 126 are provided as control data. The set of inter-scan time intervals 126 are then used to control a different or particular piece of equipment of the magnetic resonance imaging system.

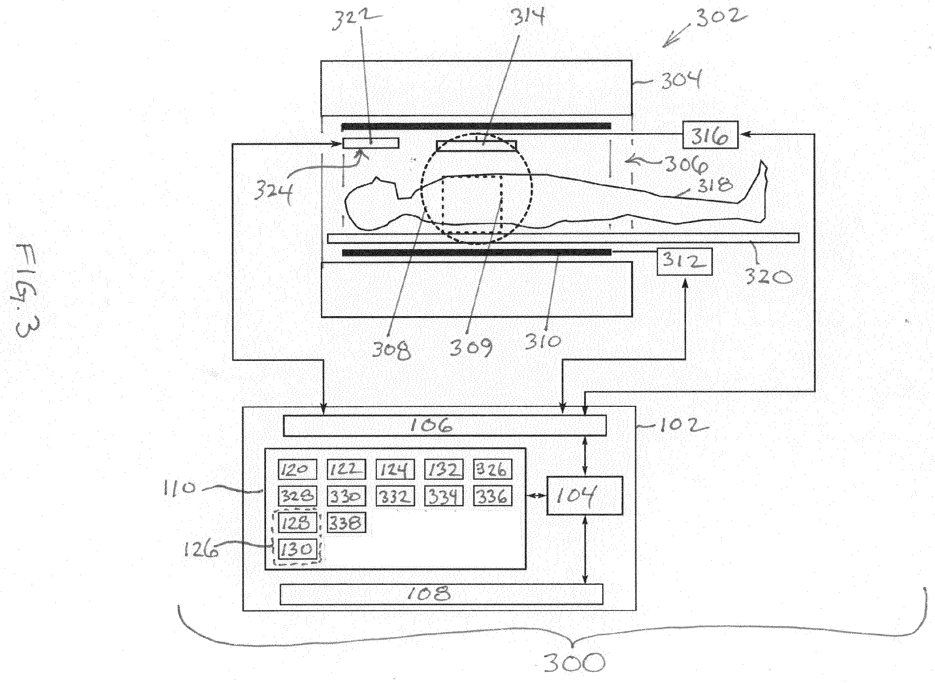

Fig. 3 illustrates a further example of a medical system 300. The medical system 300 is similar to the medical system 100 depicted in Fig. 1 except that it additionally comprises a magnetic resonance imaging system 302.

The magnetic resonance imaging system 302 comprises a magnet 304. The magnet 304 is a superconducting cylindrical type of magnet with a bore 306 through it. It is also possible to use both a split cylindrical magnet and a so-called open magnet. A split cylindrical magnet is similar to a standard cylindrical magnet, except that the cryostat has been split into two sections to allow access to the iso-plane of the magnet. Such magnets may for instance be used in conjunction with charged particle beam therapy. An open magnet has two magnet sections, one above the other with a space in-between that is large enough to receive a subject. The arrangement of the two magnet sections is similar to that of a Helmholtz coil. Open magnets are popular because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data are acquired for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used for the acquisition of k-space data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatial encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio frequency coil 314 is connected to a radio frequency transceiver 316. The radio frequency coil 314 and radio frequency transceiver 316 may be replaced by separate transmit and receive coils and a separate transmitter and receiver. It is understood that the radio frequency coil 314 and the radio frequency transceiver 316 are representative. The radio frequency coil 314 is intended to also represent a dedicated transmit antenna and a dedicated receive antenna. Likewise, the transceiver 316 may also represent a separate transmitter and receiver. The radio frequency coil 314 may also have multiple receive/transmit elements and the radio frequency transceiver 316 may have multiple receive/transmit channels.

The magnetic resonance imaging system 302 is further shown as comprising a subject display 322 which is positioned above the subject 318 so that an inter-scan progress indicator 324 is visible to the subject 318. Viewing the inter-scan progress indicator 324 during a waiting period between two of the magnetic resonance imaging scans may have a calming effect and reduce the inherent stress of having the magnetic resonance imaging procedure performed. The subject display 322, the transceiver 316 and the gradient coil power supply 312 are shown as being connected to the hardware interface 106 of the computer system 102.

The memory 110 is further shown as containing a sequence of pulse sequence commands 326 that correspond to the sequence of magnetic resonance imaging scans 122. For each of the magnetic resonance imaging scans there may be one or more pulse sequence commands that are executed and control the magnetic resonance imaging 302 to control k-space data. The memory 110 is further shown as containing multiple groups of k-space data that were acquired by the magnetic resonance imaging system 302 by controlling it with the sequence of pulse sequence commands 326. During a pause between executing two of the pulse sequence commands the inter-scan progress indicator 324 is displayed to calm the subject 318.

The memory 110 is further shown as containing a number of optional features. The memory 110 is shown as containing an optional total examination time 330, which was determined by using the duration of the sequence of magnetic resonance imaging scans 122 and the time intervals 128. It may also be determined using the confidence levels 130. This total examination time 330 was input into a scheduling application 322 which then provided a calendar file 334, which would automatically add an appointment to a calendar application. For example, the calendar file may be a so-called star.ics file.

The memory 110 is also shown as containing an optional cumulative time 336. In some examples the cumulative time 336 may be the same or equivalent to the total examination time 330. The memory 110 is further shown as containing an optional media database 338, which contains various media files such as audio or video which can be displayed or played back using the subject display 332. Using the cumulative time 336 the media database 338 selects media content 340 which matches the cumulative time 336. This may also have a calming effect on the psychology of the subject 318.

The memory 110 is further shown as containing an optional magnetic resonance image or images 342 that were reconstructed from the multiple groups of k-space data 228. This however is optional because many times the magnetic resonance image 342 may be reconstructed using a cloud-based or remote server.

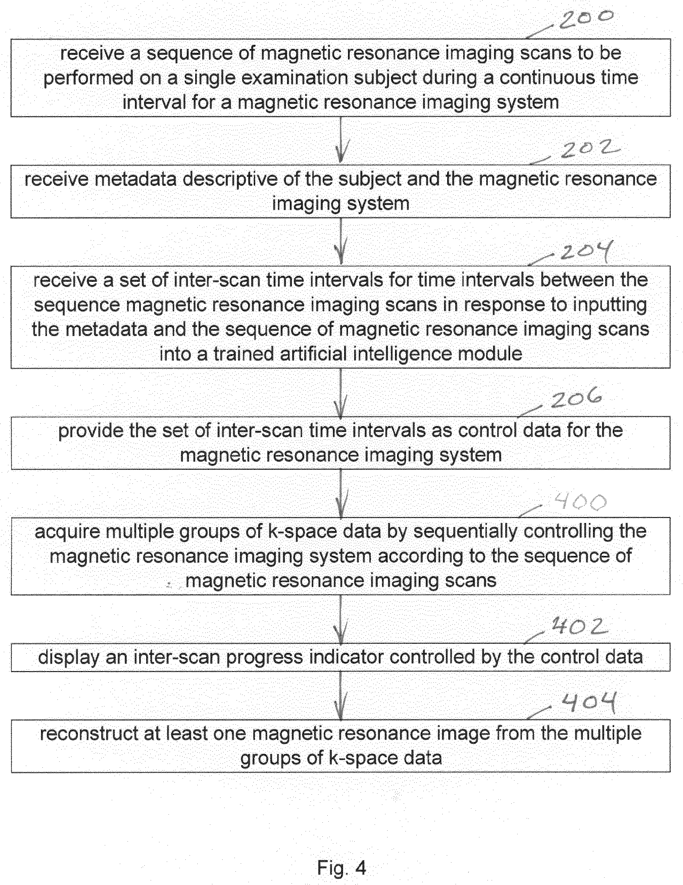

Fig. 4 shows a flowchart which illustrates a method of operating the medical system 300 of Fig. 3. Steps 200, 202, 204, and 206 are performed as was illustrated in Fig. 2. In step 400 the multiple groups of k-space data 228 are acquired by sequentially controlling the magnetic resonance imaging system according to the sequence of magnetic resonance imaging scans 122. This may for example involve using the sequence of pulse sequence commands 326. In step 402, the inter-scan progress indicator 324 is displayed to the subject 118 and is controlled by the control data 126, which is also known as the set of inter-scan time intervals. The system may alternate between acquiring k-space data and displaying this inter-scan progress indicator. In step 404, at least one magnetic resonance image 342 is reconstructed using the multiple groups of k-space data 228.

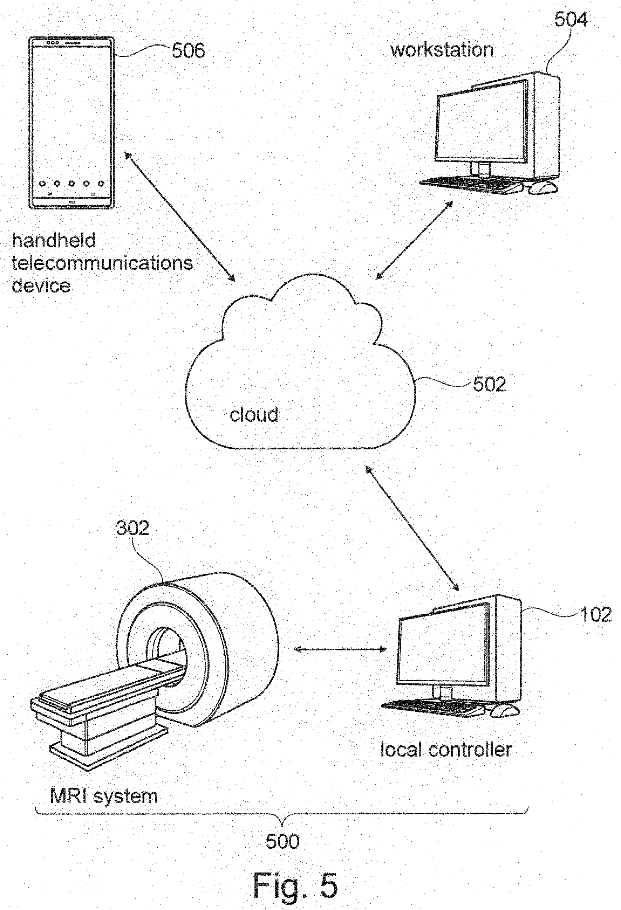

Fig. 5 illustrates a further example of a medical system 500. In this example, the various computational features of the computer 102 or local controller, as illustrated in Figs. 1 and 3, is distributed across a cloud-based computing system. The magnetic resonance imaging system 302 is shown as being controlled by a local controller. The local controller 102 would for example use the sequence of pulse sequence commands 326 to acquire the multiple groups of k-space data 228. The other functionality could be distributed via the internet or other networking system to a cloud-based computing system 502, a workstation 504, or a handheld telecommunications device 506. The cloud-based system 502 may for example provide virtual machines or remote servers which can provide image reconstruction or host the trained artificial intelligence module 132. Likewise, the same functionality can be distributed to the workstation 504, which may for example be a workstation in a radiology or other medical setting and the telecommunications device 506. For example, the handheld telecommunications device 506 may be a smartphone used by an operator or a radiologist.

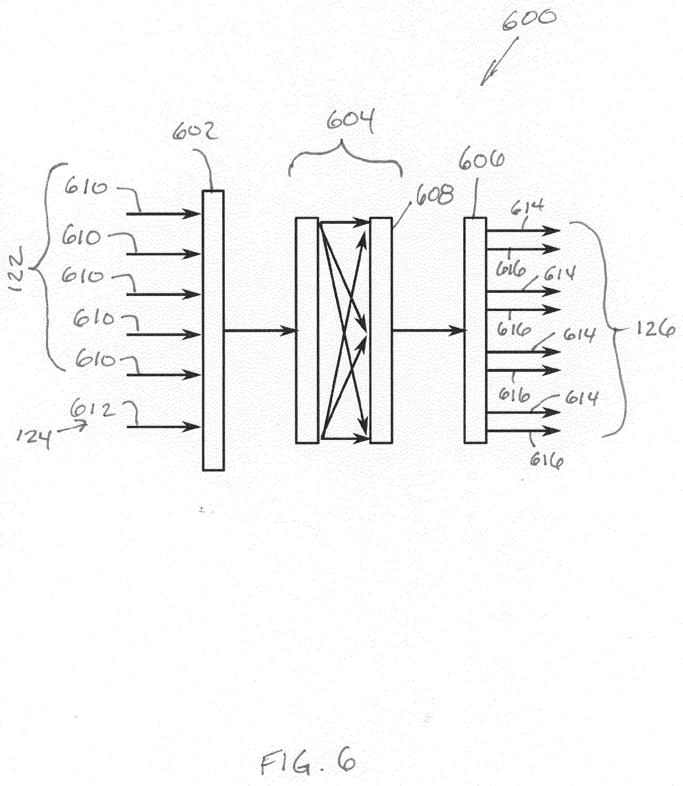

Fig. 6 illustrates an example of a neural network 600. The neural network 600 may be used as a trained artificial intelligence module 132. The neural network 600 comprises an input layer 602 that is connected to multiple fully connected layers 604. There is an output layer 606 that is connected to a last layer 608 of the fully connected layers 604. At the input layer 602, there are inputs for encoding a choice of a magnetic resonance imaging scan 610. Collectively these are used to input the sequence of magnetic resonance imaging scans 122. Additionally, there is an input 612 or multiple inputs for encoding the metadata 124. At the output layer 606, there are pairs of outputs that output the encoding for a time interval 614 and for outputting its corresponding confidence interval 616. Collectively these are then configured for outputting the set of inter-scan time intervals 126.

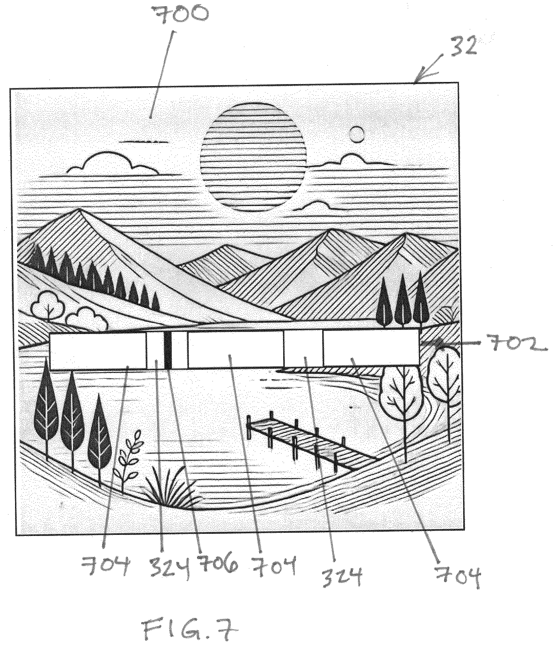

Fig. 7 illustrates and example of a subject display 322. There is a psychologically soothing image of nature 700 and a total progress indicator 702. The total scan progress indicator 702 comprises three magnetic resonance imaging scan progress indicators 704 and two inter-scan progress indicators 324. With this total progress indicator 702 the subject 318 is aware of the total time remaining as well as the expected delay between scans as indicated by the inter-scan progress indicator 324. There is a current time cursor 706 that is currently indicating the progress in one of the inter-scan progress indicators 324. A media file could be displayed such as a cartoon or movie could be displayed in place of the image 700.

Other types of displays 322 could be used in place of what is depicted in Fig. 7. This same information can be communicated to the subject 318 in alternative ways. These alternatives may also be used to communicate the uncertainty (confidence levels 130) in the time intervals 128. For example 3 parallel progress bars, showing the current time position for a 90%, 50%, 10%-quantile of earlier comparable scans could be used. In another example, curved trajectories, visualizing shorter and longer tracks to reach the same finish line (similar to circular inner vs outer race tracks for running competitions), with highlighting of the currently estimated track. Child-appropriate visualization using e.g. hares and turtles towards a common goal to illustrate the variability of the current estimate.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit", "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational systems to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the C programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a Local Area Network (LAN) or a Wide Area Network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special-purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.
A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.
A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, WLAN connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, television screen, touch screen, tactile electronic display, Braille screen, Cathode Ray Tube (CRT), storage tube, bi-stable display, electronic paper, vector display, flat panel display, Vacuum Fluorescent (VD) display, Light-Emitting Diode (LED) displays, Electro-Luminescent Display (ELD), Plasma Display Panels (PDP), Liquid Crystal Display (LCD), Organic Light-Emitting Diode (OLED) displays, a projector, and head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: optional hardware / network interface
- 108: optional user interface
- 110: memory
- 120: machine executable instructions
- 122: sequence of magnetic resonance imaging scans
- 124: metadata
- 126: set of inter-scan time intervals (control data)
- 128: time intervals
- 130: confidence levels
- 132: trained artificial intelligence module
- 200: receiving a sequence of magnetic resonance imaging scans to be performed on a single examination subject during a continuous time interval for a magnetic resonance imaging system
- 202: receiving metadata descriptive of the subject and the magnetic resonance imaging system
- 204: receiving a set of inter-scan time intervals for time intervals between the sequence magnetic resonance imaging scans in response to inputting the metadata and the sequence of magnetic resonance imaging scans into a trained artificial intelligence module
- 206: providing the set of inter-scan time intervals as control data for the magnetic resonance imaging system
- 300: medical system
- 302: magnetic resonance imaging system
- 304: magnet
- 306: bore of magnet
- 308: imaging zone
- 309: field of view
- 310: magnetic field gradient coils
- 312: magnetic field gradient coil power supply
- 314: radio frequency coil
- 316: transceiver
- 318: subject
- 320: subject support
- 322: subject display
- 324: inter-scan progress indicator
- 326: sequence of pulse sequence commands corresponding to sequence of magnetic resonance imaging scans
- 328: multiple groups of k-space data
- 330: total examination time
- 332: scheduling application
- 334: calendar file
- 336: cumulative time
- 338: media database
- 340: media content
- 342: magnetic resonance image
- 400: acquire multiple groups of k-space data by sequentially controlling the magnetic resonance imaging system according to the sequence of magnetic resonance imaging scans
- 402: display an inter-scan progress indicator controlled by the control data
- 404: reconstruct at least one magnetic resonance image from the multiple groups of k-space data
- 500: medical system
- 502: cloud computing system
- 504: workstation
- 506: handheld telecommunications device
- 600: neural network
- 602: input layer
- 604: multiple fully connected layers
- 606: output layer
- 608: last layer of the fully connected layers
- 610: input for encoding a choice of a magnetic resonance imaging scan
- 612: input for encoding metadata
- 614: output for encoding a time interval
- 616: output for encoding confidence interval
- 700: picture or video
- 702: total progress indicator
- 704: magnetic resonance imaging scan progress indicators
- 706: current time cursor

## Claims

1. A method of magnetic resonance imaging, wherein the method comprises:
- receiving (200) a sequence of magnetic resonance imaging scans (122) to be performed on a single examination subject (318) during a continuous time interval for a magnetic resonance imaging system (302);
- receiving (202) metadata (124) descriptive of the single examination subject and the magnetic resonance imaging system;
- receiving (204) a set of inter-scan time intervals (126) for time intervals between the sequence of magnetic resonance imaging scans in response to inputting the metadata and the sequence of magnetic resonance imaging scans into a trained artificial intelligence module (132), wherein the set of inter-scan time intervals comprises: paired time intervals (128) and confidence levels (130) of the time intervals;
- providing (206) the set of inter-scan time intervals as control data for the magnetic resonance imaging system.

2. The method of claim 1, wherein the method further comprises:
- acquiring multiple groups of k-space data (328) by sequentially controlling the magnetic resonance imaging system according to the sequence of magnetic resonance imaging scans; and
- displaying an inter-scan progress indicator (324) controlled by the control data, wherein the inter-scan progress indicator is configured for displaying a time tracker of a current inter-scan time interval of the set of inter-scan time intervals to the single examination subject using a subject display, wherein the inter-scan progress indicator is modified using the confidence level of the current inter-scan time interval.

3. The method of claim 2, wherein the inter-scan progress indicator is modified using the confidence level of the current inter-scan time interval by any one of the following:
- increasing the duration of the current inter-scan time interval; and
- displaying the inter-scan progress indicator with an additional progress indicator, wherein the additional progress indicator comprises an additional time buffer scaled according to the confidence level of the current inter-scan time interval, wherein the additional progress indicator is configured to perform a countdown after the inter-scan progress indicator has finished the countdown.

4. The method of claim 2 or 3, wherein the method further comprises monitoring a status of the magnetic resonance imaging system during performance of the sequence of magnetic resonance imaging scans.

5. The method of claim 4, wherein the method further comprises:
- detecting a delay during performance of the sequence of magnetic resonance imaging scans using the status of the magnetic resonance imaging system;
- updating the metadata using the detected time interval;
- updating the set of inter-scan time intervals by inputting the updated metadata and the sequence of magnetic resonance imaging scans into a trained artificial intelligence module;
- updating the inter-scan progress indicator with the updated set of inter-scan time intervals.

6. The method of claim 4 or 5, wherein the method further comprises:
- determining a set of actual inter-scan time intervals using the status of the magnetic resonance imaging system;
- construct training data using the sequence of magnetic resonance imaging scans and the metadata as trial data and using the set of actual inter-scan time intervals as ground truth data; and
- training the trained artificial intelligence module using the training data further.

7. The method of claim 4, 5, or 6, the monitoring of the magnetic resonance imaging system being performed by any one of the following:
- using an audio system to monitor gradient noise of the magnetic resonance imaging system;
- monitoring a push to talk control on an intercom system;
- using the audio system to monitor communication of the designated operator;
- determining the status using a local controller of the magnetic resonance imaging system;
- determining the status using a screen capture of a display of the local controller;
- monitoring clicks on a user interface of the magnetic resonance imaging system;-
determining the status using a video feed of the magnetic resonance imaging system and/or a control room of the magnetic resonance imaging system; and
- combinations thereof.

8. The method of any one of the preceding claims, wherein the trained artificial intelligence module comprises:
- an input layer (602) configured to receive encoded input data (610, 612) descriptive of the sequence of magnetic resonance imaging scans and the metadata;
- multiple fully connected layers (604) connected to the input layer; and
- an output layer (606) connected to a last layer(608) of the multiple fully connected layers, wherein the output layer is configured to output encoded output data (614, 616) descriptive of the set of inter-scan time intervals.

9. The method of any one of the preceding claims, wherein the trained artificial intelligence module comprises any one of the following: a large language model, recurrent neural network with a long short term memory, a statistical machine learning model, a transformer model, a machine learning model, support vector regressor, stochastic gradient descent, gradient boosted trees, and combination thereof.

10. The method of any one of the preceding claims, wherein the metadata further comprises any one of the following: a designated operator of the magnetic resonance imaging system, a clinic identifier, a department identifier, clicks entered on a control interface of the magnetic resonance imaging system, sensor data, camera data, modifications made to pulse sequences, and combinations thereof.

11. The method of any one of the preceding claims, wherein the method further comprises:
- determine a total examination time (330) using the sequence of magnetic resonance imaging scans and the control data, wherein the control data contributes the inter-can time intervals to the total examination time, and wherein the total examination time is adjusted further using the confidence levels of inter-scan time intervals; and
- scheduling the continuous time interval for the total examination time using a scheduling application (332).

12. The method of any one of the preceding claims, wherein the method further comprises:
- determine a cumulative time (336) for the sequence of magnetic resonance imaging scans using the inter-scan time intervals of the set of inter-scan time intervals;
- determine a cumulative confidence level using the confidence levels of the inter-scan time intervals;
- selecting media content (340) from a media database (338) to fill the cumulative time using the cumulative time and the cumulative confidence level; and
- providing the media content to the single examination subject during performance of the sequence of magnetic resonance imaging scans using a subject media player.

13. A computer program product comprising a computer-readable storage medium (110) having machine executable instructions (120) embodied therewith, said machine executable instructions are configured to implement the method of any one of claims 1 through 12.

14. A medical system (100, 300, 500), wherein the magnetic resonance imaging system comprises:
- a memory (110) storing machine executable instructions (120) and a trained artificial intelligence module (132);
- a computational system (104), wherein execution of the machine executable instructions causes the computational system to:
- receive (200) a sequence of magnetic resonance imaging scans (122) to be performed on a single examination subject (318) during a continuous time interval for a magnetic resonance imaging system (302);
- receive (202) metadata descriptive of the single examination subject and the magnetic resonance imaging system;
- receive (204) a set of inter-scan time intervals (126) for time intervals between the scans of the sequence of magnetic resonance imaging scans in response to inputting the metadata and the sequence of magnetic resonance imaging scans into a trained artificial intelligence module (132), wherein the set of inter-scan time intervals comprises: paired time intervals (128) and confidence levels (130) of the time intervals;
- provide (206) the set of inter-scan time intervals as control data for the magnetic resonance imaging system.

15. The medical system of claim 14, wherein the medical system further comprises:
- the magnetic resonance imaging system (302);
- a subject display (322) configured to display an inter-scan progress indicator (324) to the single exam subject;
wherein execution of the machine executable instructions further causes the computational system to:
- acquire (400) multiple groups of k-space data (328) by sequentially controlling the magnetic resonance imaging system according to the sequence of magnetic resonance imaging scans;
- display (402) an inter-scan progress indicator (324) controlled by the control data,
wherein the inter-scan progress indicator is configured for displaying a time tracker of a current inter-scan time interval of the set of inter-scan time intervals to the single examination subject using the subject display, wherein the inter-scan progress indicator is modified using the confidence level of the current inter-scan time interval;
- reconstruct (404) at least one magnetic resonance image (342) from the multiple groups of k-space data.
